# EUROPEAN PATENT APPLICATION

(11) **EP 1 736 201 A1**
(43) Date of publication of application: **27.12.2006**
(21) Application number: 05720815.9
(22) Date of filing: 15.03.2005
(51) Int. Cl.: A61N 1/00, A44C 9/02, A61N 5/06

(54) **RING WITH HEALTH PROMOTING CAPABILITY**

(30) Priority: 29.03.2004 JP 2004096431
(71) Applicant: Phild Co., Ltd., Kyoto pref. 604-8152 (JP)
(72) Inventor: HIRATA, Yoshihiro, C/O PHILD CO., LTD., Nakagyo ku, Kyoto city, Kyoto 6048152 (JP); UEDA, Yoshio, C/O PHILD CO., LTD., Nakagyo ku, Kyoto city, Kyoto 6048152 (JP); TAKASE, Hiroaki, C/O PHILD CO., LTD., Nakagyo ku, Kyoto city, Kyoto 6048152 (JP); SUZUKI, Kazuaki, C/O PHILD CO., LTD., Nakagyo ku, Kyoto city, Kyoto 6048152 (JP); SASAKI, Kazuma, C/O PHILD CO., LTD.,, Nakagyo ku, Kyoto city, Kyoto 6048152 (JP); MATSUI, Takanobu, C/O PHILD CO., LTD., Nakagyo ku, Kyoto city, Kyoto 6048152 (JP)
(74) Representative: Vossius & Partner
(86) International application number: PCT/JP2005/004560
(87) International publication number: WO 2005/092429

(57) **Abstract**

A ring having health promoting functions, which fits all fingers and comprises a fixed part, which is formed from a precious metal or titanium material and is comprised of a simple substance composition or a resin mixture composition of a simple metal or alloy powder, and an elastic flexible part.

## Description

### Field of the Invention

The present invention relates to a ring which has health promoting functions and fits all fingers.

Further, the present invention relates to a ring having health promoting functions, which is formed from a composition of a simple substance, an alloy, or a compound of Au, Ag, Pt, Pd, or titanium and comprises a fixed part in which a magnet is appropriately embedded and a flexible part.

### Background Technology

Recently, owing to increasing health consciousness, health ornaments have been widely used and the market has been inundated with products claiming to promote health or cure various maladies. Examples of these products include clothing such as underwear, ornaments such as bracelets and rings, bedding, footwear, step-on-type health devices or instruments, foods and beverages, and various exercise goods, which are used in daily life, and their demand is expected to keep increasing in years ahead.

Also there have been products which reportedly fulfill functions to promote health or cure maladies by utilizing lines of magnetic force generated by magnets (magnetism), far-infrared radiation or electromagnetic waves generated by ceramics, elemental materials such as carbon and germanium, raw materials such as natural minerals, gold/silver foils, functional waters such as α-water, and tourmaline.

However, some of these products apparently lack effectiveness in promoting health or curing maladies or fail to substantiate such effectiveness. Further, various problems have been pointed out in some products, such as unsatisfactory efficacy, slow action, relatively short duration of effectiveness, limited ranges of curing effectiveness, side effects such as cutaneous allergic reactions, unpleasant feeling when worn, costly raw materials, and cumbersomeness in use.

Precious metals such as Au, Ag, and Pt and metallic titanium are metals, whose functions have been found relatively recently as compared to iron and copper which have been used since ancient times, and are now used in many fields because of their light weight, excellent strength at high temperatures, and corrosion resistance. Recently, metallic titanium has drawn attention for use because of its physiological actions such as promotion of blood circulation and electromagnetism. Accordingly, utilization of precious metals and titanium as products for promoting health and curing maladies has been anticipated to solve the abovementioned problems.

Use of precious metals in ornaments has been known since ancient times and technology for use of titanium in ornaments and goods for daily use has been also known. For example, Japanese Patent Application Laid-open No. H8-322695 discloses a bedding to improve physical conditions during sleep using porous ceramic containing one element selected from the group including titanium; however, titanium has been described only as one of the elements and not used as a new health promoting material. Further, Registered Japanese Utility Model No. 3045835 has disclosed a health band with multiple-layer structure consisting of top and bottom layers and an intermediate layer in which a new titanium raw material such as simple titanium or a titanium compound is dispersed. This health band has a simple structure and is wrapped around the wrist or ankle in use and thereby improves health as the titanium raw material promotes blood circulation and metabolism. Also the band has been shown to generate no allergic reaction to metallic substances, such as eczema and itchiness, since the titanium raw material has no direct contact with the skin.

Further, Registered Japanese Utility Model No. 3061466 has disclosed health slippers, each pair of which comprises a sole-shaped core material on which an acupressure point applying product having curves or projections in the acupoint reflected area of the sole is fixed and a colored patterned fabric glued onto the surface of the acupressure point applying product using an adhesive which contains a titanium raw material such as simple titanium and a titanium compound. The outer edges of the slippers are raised to accommodate the entire foot up to the heel. This slipper product claims that blood circulation of the sole is improved by the stimulation of the acupressure points by the curves or projections as well as the stimulation by titanium and that its production cost is low; however, the product has not been satisfactory in terms of effectiveness.

Further, Japanese Patent Application Laid-open No. H11-285543 has disclosed a health maintenance device, in which a semiconductor film is formed on the surface of a partially reduced sintered body of titanium oxide. Registered Japanese Utility Model No. 3068810 has disclosed a health ornament in the form of a stylish fashion ring with health promoting functions, in which a ring or bracelet is formed by vulcanization by adding a mixture of amber powder and titanium oxide powder to a rubber forming material and a ring-shaped unit colored with a luminescent paint is fit into its outer groove.

Application of new metallic titanium materials in ornaments or goods for daily use using these known technologies makes good use of characteristics of metallic titanium including physiological activities such as blood circulation promotion and electromagnetism to provide improved health promoting functions as compared to conventional health ornaments; however, these health ornaments have not always been satisfactory in terms of function and comfort when worn. Further these health ornaments could not satisfy consumers' needs in terms of their efficacy.

Further, the size of finger rings is generally assigned by a number so that users have to choose the number suitable to their finger to put on. In case where the finger to put on is changed, the ring size has to be adjusted by a professional or a new ring with a suited size has to be purchased. Alteration of the ring size or purchasing a new ring is costly. A ring which fits all fingers is necessary for health improvement and thus a ring with an adjustable diameter has been desired.

An example of the ring with an adjustable diameter is a ring for sewing, which has a break in the annulus, and the ring diameter is adjusted by changing the width of the break. However, this type of conventional rings look unattractive showing the break and in the case where the rings are made of metal, repeated deformation for changing the ring diameter disadvantageously accelerates metal fatigue and the ring becomes in poor shape.

In Japanese Patent Application Laid-open No. H10-165210, a flexible adjusting system is formed on a base on which an ornament is fixed, in which multiple holes are made on one end of the ring annulus to freely hook and unhook projections made on the other end of the ring annulus. However, in this system, the alteration of the diameter of the ring is discontinuous and a suitable diameter cannot necessarily be obtained.

Further, Registered Japanese Utility Model No. 3029661 has disclosed a flexible ring, in which a pulling spring is installed inside a semicircularly formed supporting member and a chain-type member is fixed on both ends of the spring to form the ring. However, the structure of the ring is complicated and prevents stable continuous use.
Patent Reference No. 1: Japanese Patent Application Laid-open No. H08-322695
Patent Reference No. 2: Japanese Patent Application Laid-open No. H11-285543
Patent Reference No. 3: Japanese Patent Application Laid-open No. H10-165210
Patent Reference No. 4: Registered Japanese Utility Model No. 3045835
Patent Reference No. 5: Registered Japanese Utility Model No. 3061466
Patent Reference No. 6: Registered Japanese Utility Model No. 3068810
Patent Reference No. 7: Registered Japanese Utility Model No. 3029661

### DISCLOSURE OF THE INVENTION

### Problems that the Invention Attempts to Solve

Accordingly, an objective of the present invention is to provide a novel health promoting body which utilizes the abovementioned characteristics and physiological functions of titanium or a precious metal selected from Au, Ag, Pt, and Pd.

The present invention is accordingly to provide a ring comprising a titanium-based health promoting body which fits all fingers and has health promoting functions.

Metals to be used in the present invention are chemically stable as a raw material without undergoing deterioration or degeneration with time. In particular, effectiveness of titanium in promoting health and curing maladies has been recognized only recently and its actions, for example, in improving blood circulation, promoting metabolism and activating blood or cell tissues have drawn attention. Electromagnetism or the like of titanium is suggested to affect acupoints of the whole body. Further, these actions are effective without direct contact of titanium with the skin and generated relatively fast and the effectiveness lasts for a long period of time. It has been also reported that owing to the abovementioned actions, health promoting effects become marked and affect on curing all sorts of maladies in the body, effectively curing general fatigue and debilities and ailments such as headache and backache. However, logical reasons for these unexplainable effectivenesses have not been satisfactorily elucidated; presumably, extremely mild electric action (a trace of electric current) or magnetic action may activate iron atoms in hemoglobin in the blood or a trace of electromagnetic wave of titanium may affect protein molecules in cell tissues, but these are merely speculative.

### Means to Solve the Problems

The present invention is characterized by a ring which fits all fingers, basically comprising the following components.
(1) A ring which has health promoting functions and fits all fingers, characterized by comprising a fixed part, which is composed of a single substance compact or a resin mixture composition molded article of a metallic powder, and an elastic flexible part.
(2) The ring according to (1), characterized in that said metal is a metal selected from a simple substance, an alloy or a compound of Au, Ag, Pt, Pd, or titanium.
(3) The ring according to (1) or (2), characterized in that a powdered titanium is used, which is obtained by burning a gaseous oxygen-hydrogen mixture in high-pressure water and heating metallic titanium using the resultant burning gas.
(4) The ring according to (1), (2), or (3), characterized in that a magnet is embedded in said fixed part.

### BRIEF DESCRIPTION OF THE DRAWINGS

Fig. 1 is a perspective view of a ring according to the present invention.
Fig. 2 illustrates exploded views in perspective of a fixed part and a flexible part.
Fig. 3 is a perspective view illustrating assembly of a flexible part with a spring rod-like member generally used for supporting a watch with a watchband (hereinafter referred to as "a spring rod-like member for watches").
Fig. 4 is a sectional view illustrating a magnet embedded in a fixed part.
Fig. 5 illustrates a ring having health promoting functions fitting onto a finger.

### DESCRIPTION OF THE SYMBOLS

1. Heath promoting body (fixed part)
2. Flexible part (elastic fabric)
3. Spring-like member for watches
4. Hole for inserting a spring rod-like member for watches
5. Magnet

### BEST MODE FOR CARRYING OUT THE INVENTION

Examples of the material for the health promoting body include simple metals such as Au, Pt, Ag, and Pd, their alloys such as Au-Ge, Au-Cu, Ag-Pd, Pt-Cu, and Pt-Pd, simple metallic titanium, and titanium alloys, as well as any compounds containing the abovementioned metallic atoms and titanium atoms. For example, in the case of titanium, minerals such as rutile, brookite, and octahedrite can be used. As titanium alloys, alloys of titanium with metals such as copper, tin, iron, aluminium, chromium, cobalt, molybdenum, and tungsten can be used. Examples of the titanium alloys include Ti-Al, Ti-V, Ti-Mo, Ti-Cr, Ti-Mn, Ti-Fe, Ti-Al-Cr, and Ti-Cr-Fe-O; the ratio of the components in each alloy can be appropriately selected.

Further, examples of the titanium compounds include hydrides such as TiH₂ and TiH₄; oxides such as TiO, Ti₂O₃, TiO₂, Ti(OH)₂, Ti(OH)₃, and M₂TiO₃ (M is a metal atom); sulfides such as TiS, Ti₂S₃, and TiS₂; oxyacid salts such as Ti₂(SO₄)₃, Ti(SO₄)₂, and TiP₂O₇; boron compounds such as Ti₂B, TiB, TiB₂, and Ti₂B₅; titanium carbide (TiC); silicon compounds such as TiSi₂, TiSi, and Ti₅Si₃; nitrides such as TiN, Ti₃N₄, Ti₃N₆, and Ti₅N₆; phosphorus compounds such as TiPn; titanium halides such as TiCl₂, TiCl₃, TiCl₄, TiBr₂, TiBr₃, TiBr₄, TiI₂, TiI₃, and TiI₄; complex salts such as M₂TiF₅, M₃TiF₆, M₂TiF₆, M₂[TiCl₅(OH)₂], M₂TiCl₆, [Ti(OH)₆]Cl₃, and M₂[TiBr₆]; and titanates such as CaTiO₃, SrTiO₃, BaTiO₃, CdTiO₃, and PbTiO₃. These compounds are used alone or in combination. Of titanium powders and titanium alloy powders, it is particularly appropriate to use a titanium powder manufactured by burning a gaseous oxygen-hydrogen mixture in high-pressure water and heating metallic titanium using the resultant burning gas to reduce it to powder (Japanese Patent No. 3507063).

On the other hand, in the case where the abovementioned metals or titanium are used, they can be used by mixing them in the form of powder or alloy powder into a resin. The abovementioned titanium powder is admixed with a resin material such as polyethylene and polypropylene and the admixture is processed by compression molding or the like.

As synthetic resin materials, in addition to the abovementioned polyethylene and polypropylene, polyvinyl chloride resins, polyolefin resins such as polyesters and polypropylene, polyamide resins such as nylon, and other known resins having a high solidity at normal temperature are appropriately used. The mixing ratio of the resin material and titanium powder is not particularly limited and powder or alloy powder of Au, Ag, Pt, Pd, or titanium can be used up to about 50% by weight.

The elastic flexible part wraps a spring rod-like member for watches, which is flexible at both ends, and the overlapping part is adhered, which can be easily done with an adhesive or by heating or sewing.

A ring comprising a health promoting body with use of a titanium material will be explained below.

The ring comprises a fixed part and a flexible part made of elastic fabric, which are connected using a spring rod-like member for watches.

### Example 1

An embodiment of the present invention will be explained by referring to drawings.

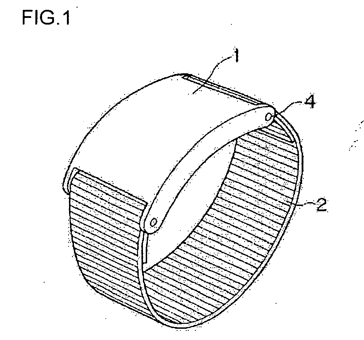

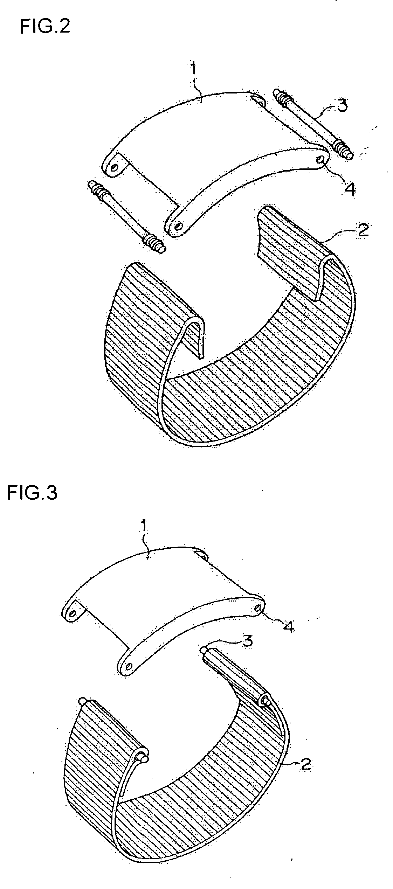

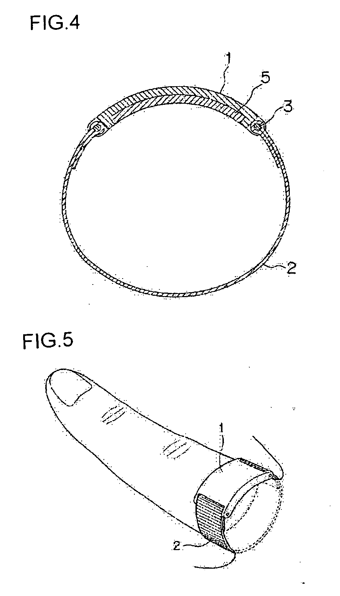

Fig. 1 is a perspective view of the present invention, Fig. 2 is exploded views in perspective of a fixed part and a flexible part, Fig. 3 is a perspective view illustrating assembly of the flexible part with a spring rod-like member for watches, Fig. 4 is a sectional view illustrating a magnet embedded in the fixed part, and Fig. 5 illustrates a ring having health promoting functions fitting onto a finger.

More specifically, the ring having health promoting functions according to the present invention is comprised of a titanium health promoting body 1, an elastic flexible part 2 and a spring rod-like member for watches 3. The spring rod-like member for watches 3 is wrapped into the elastic flexible part 2 and the edge of the flexible part is fixed by means of thermal adhesion or the like. The spring rod-like member for watches 3 fixed on the flexible part is inserted into a hole 4 made on the edge of the fixed part comprising titanium health promoting body. This spring rod-like member for watches 3 can freely be put on and off, which makes it possible to wash or exchange the flexible part if necessary.

Further, a magnet 5 can be embedded in the titanium health promoting body to further improve its effectiveness.

### Effectiveness of the Invention

A ring of the present invention has health promoting functions and fits all fingers using an elastic flexible part. Since a simple substance composition or a resin mixture composition of each powder of an alloy or a metallic compound of a metal selected from Au, Ag, Pt, and Pd, or further metallic titanium, a titanium alloy, or titanium compound is used in a fixed part, the ring can exhibit characteristic health promoting functions. Further, the elastic flexible part arranged apart from the fixed part wraps a spring rod-like member for watches, which is flexible at both ends, and the resultant overlapping parts are bonded together. The bonding can be easily done with an adhesive or by heating or sewing.

Further, the present invention relates to a ring having health promoting functions comprising a fixed part, which is composed of a composition of a simple substance, an alloy, or a compound of the abovementioned metal or titanium and appropriately has an embedded magnet, and a flexible part.

## Claims

1. A ring which has health promoting functions and fits all fingers, **characterized by** comprising a fixed part, which is composed of a single substance compact or a resin mixture composition molded article of a metallic powder, and an elastic flexible part.

2. The ring according to claim 1, **characterized in that** said metal is a metal selected from a simple substance, an alloy or a compound of Au, Ag, Pt, Pd, or titanium.

3. The ring according to claim 1 or 2, **characterized in that** a powdered titanium is used, which is obtained by burning a gaseous oxygen-hydrogen mixture in high-pressure water and heating metallic titanium using the resultant burning gas.

4. The ring according to claim 1, 2, or 3, **characterized in that** a magnet is embedded in said fixed part.
